# EUROPEAN PATENT SPECIFICATION

(11) **EP 4 093 328 B1**
(45) Date of publication and mention of the grant of the patent: **14.10.2026**
(21) Application number: 21705820.5
(22) Date of filing: 23.01.2021
(51) Int. Cl.: A61F 2/07, A61F 2/06

(54) **HYBRID STENT GRAFT AND METHODS OF BODY LUMEN REPAIR**
HYBRIDES STENTIMPLANTAT UND VERFAHREN ZUR REPARATUR EINES KÖRPERLUMENS
ENDOPROTHÈSE COUVERTE HYBRIDE ET MÉTHODES DE RÉPARATION DE LUMIÈRE CORPORELLE

(30) Priority: 24.01.2020 US 202062965321 P
(43) Date of publication of application: 30.11.2022
(73) Proprietor: The Cleveland Clinic Foundation, Cleveland, OH 44195 (US)
(72) Inventor: ROSELLI, Eric, E., Cleveland, Ohio 44195 (US)
(74) Representative: HGF
(86) International application number: PCT/US2021/014804
(87) International publication number: WO 2021/151033

(56) References cited:
- WO-A1-2005/034809
- US-A1- 2009 093 873
- US-A1- 2016 067 067
- US-A1- 2017 027 683

## Description

### Related Application

This application claims priority from U.S. Provisional Application No. 62/965,321, filed 24 January 2020.

### Technical Field

This disclosure relates to an apparatus and methods for use of a hybrid stent graft device to facilitate repair of body lumens in a subject and, more particularly, to a hybrid stent graft device comprising stations for facilitating additional interventions through the hybrid stent graft device.

### Background

A leading cause of disability and death in both the U.S. and abroad includes damage to a portion of the vascular system. This is a particular problem with regard to aortic aneurysms and dissections. Diseases of the aorta, for example, are becoming an increasing concern as a major cause of death in the U.S. and many other developed countries. Severe arterial sclerosis, severely calcified aorta, and other indications continue to necessitate complete or partial aortic replacement procedures.

Aneurysms are typically characterized by diseased or damaged blood vessels which lead to a weakening of the vessel wall. Weakening of the vessel wall can then lead to a blood-filled dilation of the vessel. Left untreated, aneurysms will eventually rupture and result in acute (and often fatal) hemorrhaging in a very short period of time.

Dissections occur when the aorta tears and layers of the aorta separate. As the layers of the aorta separate, a new lumen is formed and blood flows into this newly created lumen, which can lead to serious problems for a patient. Potential problems can include: (i) compromised blood flow to branch vessels arising from the aorta leading to organ malperfusion, ischemia, and potential disability or death (ii) growing so large that it can compress the regular opening of the aorta and cause decreased blood flow to organs. Dissections and Aneurysms are both complex diseases to treat. Treatments can include surgical options, endovascular options, and/or medicinal options. One traditional surgical and endovascular treatment approach is placing a stent graft in a vessel to allow a path for blood to flow through and protect against complications associated with the diseased aorta.

The aorta has numerous arterial branches. The arch of the thoracic aorta, for example, typically has three major branch vessels arising from the convex upper surface of the arch and ascending through the superior thoracic aperture to the root of the neck. The proximity of an aneurysm or dissection to a branch vessel may limit the use of an excluding device, such as a tubular stent graft. For example, the main body or ends of a tubular stent graft may occlude or block the branch vessels as a result of the position of the stent graft being dependent on the availability of healthy aorta segments, i.e., non-diseased or non-dilated portions of the artery wall, on the distal and/or proximal ends to ensure the stent graft is well apposed to the vessel. Sometimes it may be beneficial to block the branch vessels and other times it may be beneficial to keep the branch vessels clear. However, there may be an inadequate length of healthy tissue for the stent graft to seal against in the area of the diseased aorta and the location of the branch vessels using a traditional stent graft.

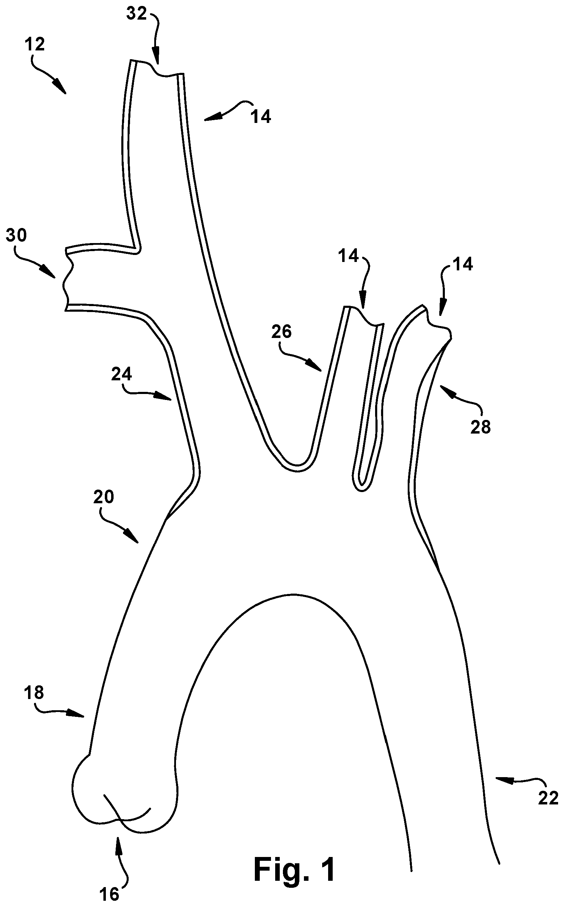

To assist the reader in understanding the relevant anatomy and physiology to which certain aspects of the present disclosure pertain, Fig. 1 illustrates a cross-sectional view of a human aortic arch 12 , also known as the transverse aorta, 12, including the aortic arch branch vessels 14. The aorta is the largest vessel in the body. It transports oxygenated blood from the left ventricle of the heart (not shown) to every organ. The aorta starts in the heart with the aortic valve 16, which is immediately adjacent the aortic root 18 and followed by the ascending aorta 20, the aortic arch (transverse aorta) 12, the descending aorta 22, and the thoracoabdominal aorta (not shown). The aorta ends in the abdomen after bifurcation of the abdominal aorta in the two common iliac arteries (not shown). The aortic arch 12 gives off the brachiocephalic trunk 24, the left common carotid artery 26, and the left subclavian artery 28. The brachiocephalic trunk 24 splits to form the right subclavian and the right common carotid arteries 30 and 32, which supply blood to the right arm and the right side of the neck and head. The left common carotid artery 26 and left subclavian artery 28 perform parallel functions on the left side. US2016/067067A1 relates to a frame structure adapted for use with a stent graft.

### Summary

The invention is defined in the appended claims.

In another aspect of the present disclosure a method of providing a stent graft to a body lumen of a subject including the following steps is described. Positioning the state graft at a target position in the body lumen. The stent graft can have an elongated body, a graft material covering, and at least one station positioned on the graft material of the elongated body. The elongated body can have proximal and distal end portions longitudinally separated by an intermediate portion and a lumen extending at least partially though the intermediate portion. The elongated body can also have a frame structure having inner and outer surfaces. At least one of the inner and outer surfaces can be at least partially covered by the graft material, wherein the proximal end portion includes an aperture in fluid communication with the lumen and defined by a portion of the frame structure. The at least one station positioned on the graft material of the elongated body can have a perimeter defined by a ring structure connected to the graft material covering the frame structure and a selectively open aperture bounded by the perimeter. The aperture of the at least one station is selectively covered by the graft material. Aligning the at least one station in the stent graft with at least one branch vessel of the body lumen. Securing the stent graft at the target position. Opening the at least one station that aligns with the at least one branch vessel to create a fenestration at the at least one station. Deploying at least one secondary stent though the at least one fenestration to connect the at least one branch vessel with the stent graft. And, securing the at least one secondary stent in the at least one branch vessel.

In another aspect of the present disclosure a method of providing a stent graft to an aortic arch of a subject, the method including the following steps is described. Providing a previously positioned stent graft within the aortic arch of the subject. The stent graft including an elongated body, a graft material covering, and a plurality of docking rings positioned on the graft material of the elongated body. The elongated body can have proximal and distal end portions longitudinally separated by an intermediate portion and a lumen extending at least partially though the intermediate portion. The elongated body can also have a frame structure having inner and outer surfaces. At least one of the inner and outer surfaces can be at least partially covered by the graft material, wherein the proximal end portion includes an aperture in fluid communication with the lumen and defined by a portion of the frame structure. The plurality of docking rings positioned on the graft material of the elongated body can each have a perimeter defined by a ring structure connected to the graft material covering the frame structure and a selectively open aperture bounded by the perimeter. The apertures of each of the plurality of docking rings are selectively covered by the graft material.

### Brief Description of the Drawings

For a better understanding, reference may be made to the accompanying drawings, in which:
Fig. 1 schematically depicts a cross section of a human aorta;
Fig. 2 schematically depicts a cross section of a stent graft;
Fig. 3 is a top view of a portion of the stent graft of Fig. 2;
Fig. 3a is a top view of a portion of the stent graft of Fig. 2;
Fig. 4a-4f depict various fenestrations of the stent graft;
Fig. 5 depicts a cross section of the stent graft with a secondary stent introduced; and
Figs. 6-11 are flow chart illustrations of methods of using a stent graft.

### Description of Aspects of the Disclosure

Unless defined otherwise, all technical and scientific terms used herein have the same meaning as is commonly understood by one of ordinary skill in the art to which the present disclosure pertains.

As used herein, the term "subject" can be used interchangeably with the term "patient" and refer to any warm-blooded organism including, but not limited to, human beings, pigs, rats, mice, dogs, goats, sheep, horses, monkeys, apes, rabbits, cattle, farm animals, livestock, etc.

As used herein, the term "user" can refer to a medical professional such as a surgeon, doctor, or nurse, or another individual qualified to make decisions regarding the structure and use of a stent graft.

As used herein, the terms "treat" or "treating" can refer to therapeutically regulating, preventing, repairing, improving, alleviating the symptoms of and/or reducing the effects of damage, diseases, or disorders on a bodily vessel, such as the aorta. As such, treatment also includes situations where a stent graft with at least one station is introduced into a body lumen and a secondary stent is introduced into a branch vessel to remedy problems with the body lumen and/or the branch vessel, such as an aneurysm or dissection, or at least a symptom associated therewith, is reduced, e.g., at least partially prevented from happening or stopped (e.g., terminated) such that the subject no longer suffers from at least a part of the damage, disease, or disorder of the body lumen, or at least the symptom(s) associated therewith.

As used herein, the singular forms "a", "an", and "the" can include the plural forms as well, unless the context clearly indicates otherwise. It will be further understood that the terms "comprises" and/or "comprising", as used herein, can specify the presence of stated features, steps, operations, elements, and/or components, but do not preclude the presence or addition of one or more other features, steps, operations, elements, components, and/or groups thereof.

As used herein, the term "and/or" can include any and all combinations of one or more of the associated listed items.

As used herein, phrases such as "between X and Y" and "between about X and Y" can be interpreted to include X and Y.

It will be understood that when an element is referred to as being "on", "attached" to, "connected" to, "coupled" with, "contacting", "adjacent", etc., another element, it can be directly on, attached to, connected to, coupled with, contacting, or adjacent the other element, or intervening elements may also be present. In contrast, when an element is referred to as being, for example, "directly on", "directly attached" to, "directly connected" to, "directly coupled" with, "directly contacting", or "directly adjacent" another element, there are no intervening elements present. It will also be appreciated by those of ordinary skill in the art that references to a structure or feature that is disposed "directly adjacent" another feature may have portions that overlap or underlie the adjacent feature, whereas a structure or feature that is disposed "adjacent" another feature might not have portions that overlap or underlie the adjacent feature.

Spatially relative terms, such as "under", "below", "lower", "over", "upper", "proximal", "distal", and the like, may be used herein for ease of description to describe one element or feature's relationship to another element(s) or feature(s) as illustrated in the figures. It will be understood that the spatially relative terms can encompass different orientations of a device in use or operation, in addition to the orientation depicted in the figures. For example, if a device in the figures is inverted, elements described as "under" or "beneath" other elements or features would then be oriented "over" the other elements or features.

As used herein, the phrase "at least one of X and Y" can be interpreted to include X, Y, or a combination of X and Y. For example, if an element is described as having at least one of X and Y, the element may, at a particular time, include X, Y, or a combination of X and Y, the selection of which could vary from time to time. In contrast, the phrase "at least one of X" can be interpreted to include one or more Xs.

As used herein, the term "covered" is defined as putting something on top of or in front of another structure, especially in order to protect, conceal, or occlude fluid flow therethrough. Covered may mean fully covered or partially covered.

As used herein, the term "open" is defined as allowing fluid communication through a space that was previously fully or partially occluded. The act of opening a structure can include, but is not limited to, puncturing, slicing, cutting into, and/or cutting out at least a portion of the structure with, a needle, scalpel, scissors, guidewire, or another mechanical tool, and/or using electricity, light, heat (e.g., electrocautery), or another energy tool (e.g., a laser, radiofrequency, etc.) to create a hole or new aperture in a covering.

It will be understood that, although the terms "first", "second", etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or steps) is not limited to the order presented in the claims or figures unless specifically indicated otherwise..

The present disclosure relates generally to vascular repair of bodily vessels and more particularly to an improved stent graft that facilitates secondary stenting and other secondary interventions in bodily vessels occluded, or partially occluded, by traditional stent grafts. The invention comprises, consists of, or consists essentially of the following features, in any combination.

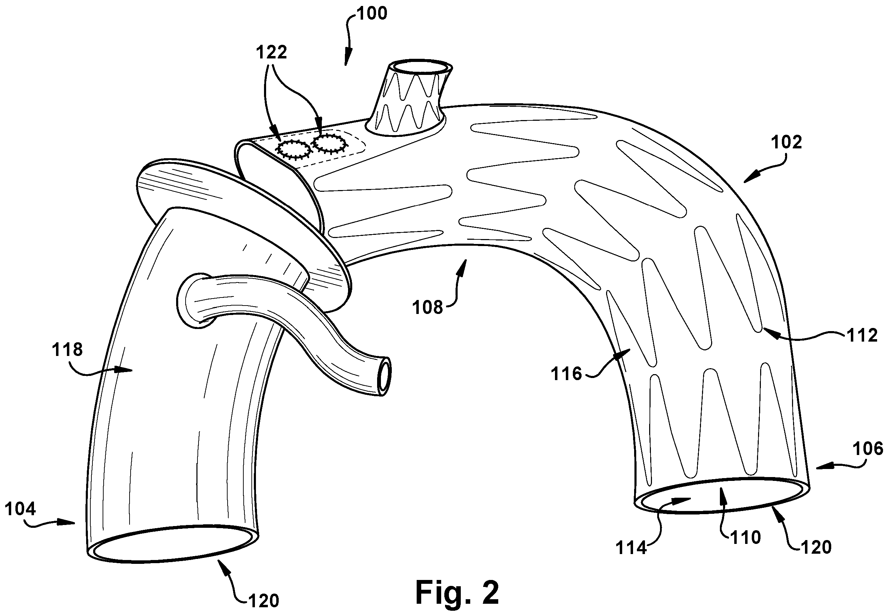

Fig. 2 depicts a cross sectional view of a stent graft 100 configured for implantation in the aortic arch 12. Other stent graft configurations, not shown, can be provided in different shapes for different body lumens, with each configuration being based on the anatomy of the specific body lumen in which that configuration is to be used. The stent graft 100 can have an elongated body 102 having a proximal end portion 104 and a distal end portion 106 longitudinally separated by an intermediate portion 108. A lumen 110 can extend at least partially through the intermediate portion 108. The lumen 110 may extend through any or all of the intermediate portion 108. In one example, the lumen 110 can extend for the entire length of the elongated body 102, allowing fluid communication with the body lumen on both ends of the stent graft.

The elongated body 102 can also include a frame structure 112 having an inner surface 114 and an outer surface 116. The frame structure 112 can be, for example, one or more of the frame structures described in U.S. Patent Application No. 14/849,785, filed 10 September 2015 and titled "Frame Structures, Stent Grafts Incorporating the Same, and Methods for Extended Aortic Repair" or U.S. Patent Application No. 16/155,176, filed 9 October 2018 and titled "Endovascular Grafts and Methods for Extended Aortic Repair". The inner surface 114 and/or the outer surface 116 can be at least partially covered by a graft material 118. The graft material 118 can cover all or a part of the inner surface 114 and/or the outer surface 116. At least one of the proximal end portion 104 and the distal end portion 106 can include an aperture 120 in fluid communication with the lumen 110. The aperture(s) 120 are defined by a portion of the frame structure 112. The aperture(s) 120 enable fluid communication between the body lumen and the lumen of the elongated body 102. The stent graft 100 also includes at least one station 122 positioned on the graft material 118 of the elongated body 102. Two stations 122 are illustrated in Figs. 2-5 by way of example, but any number of stations can be positioned in any number of configurations on the graft material 118 of the elongated body 102.

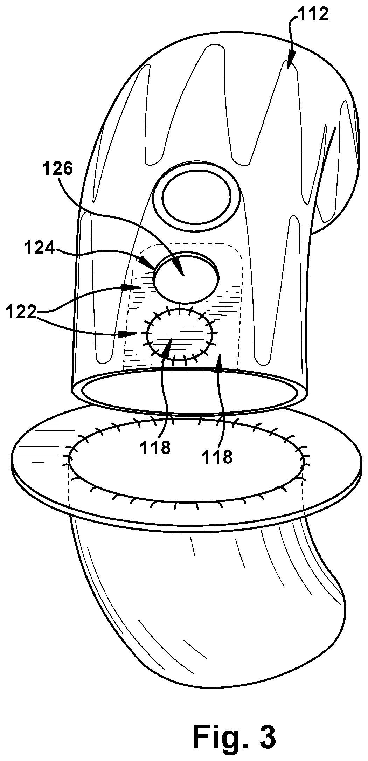

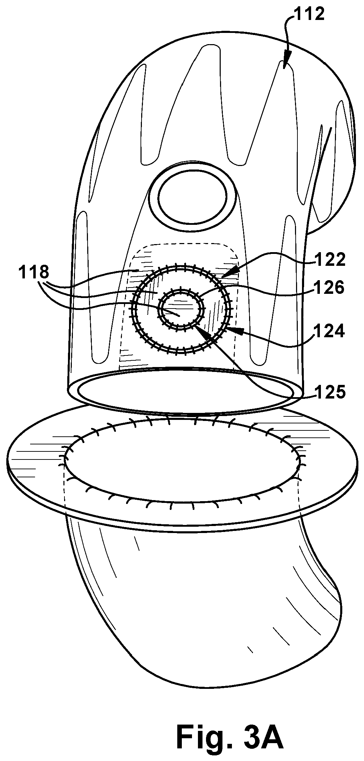

Fig. 3 depicts the at least one station 122 of stent graft 100. The at least one station 122 can have a perimeter defined by a ring structure 124 connected to the graft material 118 covering the frame structure 112. The at least one station 122 can also have a selectively open aperture 126 bounded by the perimeter. The aperture 126 of the at least one station122 is selectively covered by the graft material 118. The terms "selectively open" and "selectively covered" are used herein to indicate that the aperture 126 of the at least one station 122 can be completely uncovered (e.g., open), partially covered, or completely covered, and that the station 122 can be transitioned between any of those states by a user as desired for a particular use environment. It is contemplated that the covering of graft material 118 can be on top of and/or underneath the ring structure 124 defining the bounds of the aperture 126. When the aperture 126 is partially covered or completely covered by
graft material 118 at least a part of the aperture is occluded. The aperture 126 can be covered by the graft material 118 covering the elongated body 102 and a user may choose the extent to which the aperture is open or covered after the ring structure 124 is positioned on the graft material by removing a part or all of the graft material covering the aperture. It is contemplated that the aperture 126 of the at least one station 122 can also or instead be covered by an additional piece of graft material 118, separate from the graft material covering the elongated body 102, attached (e.g., sewn, adhered, etc.) to the ring structure 124 . The ring structure 124 defining the perimeter is a biocompatible material, comprised from a ring of sutures. The ring structure 124 can be attached to the graft material 118 in any suitable manner such as, but not limited to, being sewn into the graft material 118, integrated and/or interwoven into the graft material 118, and/or adhered to the graft material 118

The at least one station 122 can be positioned proximate the proximal end portion 104 of the elongated body 102, as shown in Figs. 2- 3, to substantially align with at least one branch vessel 14 of the aorta when the stent graft 100 is located in the aorta. The at least one branch vessel 14 of the aorta can be at least a selected one of the brachiocephalic (trunk) artery 24, the left common carotid artery 26, and the left subclavian artery 28. The at least one station 122 can also or instead be configured to substantially align with other branch vessels when the main body lumen is not the aortic arch 12. When the body lumen is part of the aorta it can be one of the aortic arch, the ascending aorta, the descending aorta, or the abdominal aorta. Other, non-exhaustive examples of body lumens include portions of the renal artery, the coronary artery, the carotid artery, or airways in the lungs. One of ordinary skill in the art will be readily able to provide a stent graft 100 according to the present invention for any desired use environment. Accordingly, the at least one station 122 can be positioned at any point on the elongated body 102 and if there is more than one station 122, each station 122 can be positioned anywhere on a surface of the elongated body 102, as desired for a particular use environment of the stent graft 100.

The perimeter of the at least one station 122 can have any shape, size, location on the graft, cross-sectional area, and/or other physical configuration appropriate for aligning with at least a selected branch vessel 14. The perimeter of the at least one station 122 defined by ring structure 124 is depicted in the Figures as being substantially circular. However, the shape of the perimeter can include, but is not limited to, at least one of a rectilinear shape, a curvilinear shape, a circle, an oval, a rectangle, a polygon, and/or any other desired perimeter shape for a particular use environment. When the at least one station 122 includes two or more stations, each station can have any suitable shape, size, location on the graft, perimeter footprint, cross-sectional area, and/or any other physical characteristics, similar to or different from those of the other stations 122. The at least one station 122 can include at least one secondary ring structure 125 as shown in Fig. 3A. The at least one secondary ring structure 125 can be located within the perimeter defined by the ring structure 124 of the at least one station 122. The secondary ring structure can define an aperture with a smaller area within the station 122 and can be, but is not necessarily, concentric with the ring structure 124 defining the first, outer station. The secondary ring structure 125 can be comprised of the same material as the ring structure 124 or a different material. The secondary ring structure 125 can be separately covered by the graft material 118, as shown, or the secondary ring structure can be covered by the same graft material as the ring structure 124. For example, the outer ring structure 124 can be a 10 mm diameter ring and the secondary ring structure 125 can be a 6 mm diameter ring located inside the perimeter of the first ring structure 124, where a user has an option to open only the 6 mm diameter ring for a smaller branch vessel or another option to open both the 6 mm diameter ring and the 10 mm diameter ring for a larger branch vessel. The secondary ring structure 125 can be removed and/or fractured as part of a process of opening the outer ring structure 124. For example, a balloon may be used to fracture and/or cut through a part of the ring structure 125 comprising a ring of sutures instead of removing the ring.

The at least one station 122 of the stent graft 100 facilitate the creation of a fenestration in the stent graft 100. A fenestration is an aperture or opening in a surface created via an intervention; the surface being opened can be a biological surface or a man-made surface. Preferably a fenestration is created at the at least one station after the stent graft has been positioned within a selected body lumen of a subject. However, a fenestration can also be created at the at least one station 122 before the stent graft 100 is positioned within a selected body lumen of a subject. A fenestration can be created with various techniques such as, but not limited to, using at least one of a scissors, a scalpel, a needle, a guidewire, an electric current, a laser, and/or any other device capable of creating a fenestration to puncture, cut, remove or otherwise alter any portion of the at least one station 122 to result in a fenestrated station. It is contemplated that the graft material can be fenestrated via at least one of removal, displacement, destruction (e.g., dissolution or vaporization), or any other suitable technique that results in the graft material being altered to include an aperture therethrough.

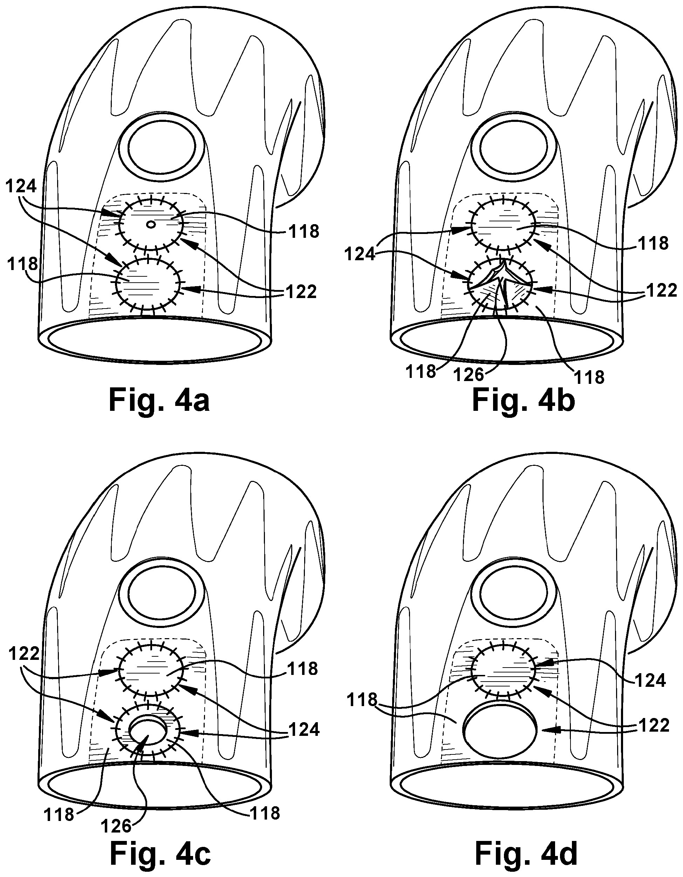

Figures 4a-4f depict examples of fenestrated stations. As shown in Figures 4a-c, a fenestration can be created by opening at least a section of the graft material 118 selectively covering the aperture 126 of the at least one station 122. Opening can include but is not limited to, puncturing a section of the graft material 118 covering the aperture 126 of the at least one station 122 (see Fig. 4a), cutting through a section of the graft material 118 covering the aperture 126 of the at least one station 122 such that flaps of the graft material 118 are still attached to the ring structure 124 (see Fig, 4b), and/or cutting out and removing a section of the graft material 118 covering the aperture 126 of the at least one station 122 such that a hole exists within the graft material 118 of the at least one station 122 (see Fig. 4c).

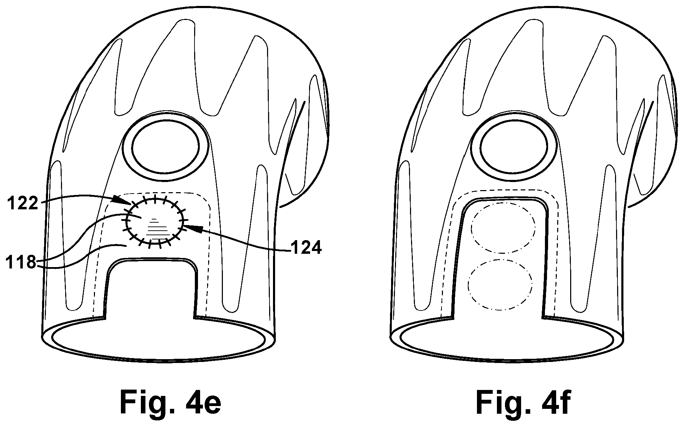

As shown in Fig. 4d, a fenestration can also be created by removing the ring structure 124 defining the perimeter of the at least one station 122, including removing the graft material 118 selectively covering the aperture 126 of that at least one station 122, thereby creating a hole with the same dimensions as the removed at least one station 122. Fig. 4e depicts a fenestration technique that includes the removal of a section of the graft material 118 of the elongated body 102 disposed between the ring structure 124 of the at least one station 122 and the proximal end portion 104 of the elongated body 102. Optionally, the at least one station 122 can also be removed at the same time such that the ring structure 124 and the graft material 118 covering at least part of the aperture 126 of the at least one station 122 are at least partially removed. The entirety of both the ring structure of the at least one station 122 and the graft material 118 selectively covering the aperture 126 of the at least one station 122 can also be removed as part of this method of fenestration.

Finally, Fig. 4f depicts a fenestration created by removing a portion of the stent graft 100 including the at least one station 122, a portion of the frame structure 112 of the elongated body 102, and the graft material 118 at least partially covering the elongated body 102. When the at least one station 122 includes more than one station 122, one of the stations may be removed or multiple stations may be removed by the above described fenestration methods. The type of fenestration at the at least one station 122 can be chosen, for example by a user, based on the damage suffered by the aorta 12 (or other body lumen) and the number and nature of the branch vessels 14 occluded by the elongated body 102 of the stent graft 100 when the stent graft 100 is positioned in the aorta (or other body lumen).

It is contemplated that a fenestration will often be created during or after introduction of the stent graft 100 into a body lumen. However, a fenestration can be created before, during, and/or after introduction of the stent graft 100 into a body lumen. In one option, one or more fenestrations could be created during a bench top procedure before the stent graft 100 is placed *in situ.* In another option, one or more fenestrations can be created during the same intervention that places the stent graft 100 within a body lumen of the subject. In an additional option, one or more fenestrations can be created after the stent graft 100 is introduced into a body lumen, in the same intervention or a later intervention. It is contemplated that one or more of these options of timing for the creation of a fenestration can be used with the same stent graft 100.

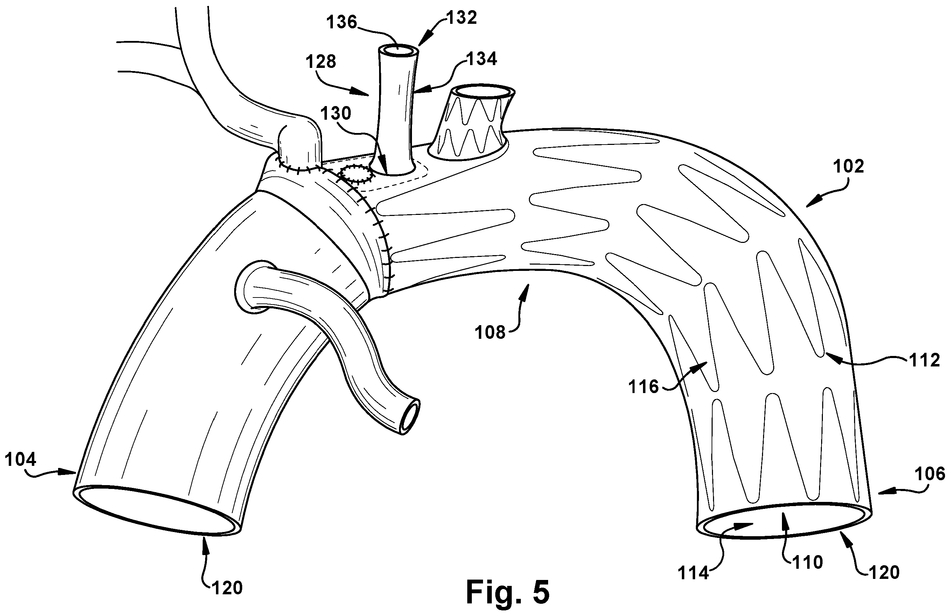

Once the stent graft 100 is fenestrated, the fenestration provides a site in the stent graft at which a secondary stent can be introduced to the at least one branch vessel aligned with at least one station. Fig. 5 depicts a stent graft 100 with a secondary stent 128 positioned at a fenestration of the at least one station 122. The secondary stent 128 can at least partially pass through the fenestration of the at least one station 122. The secondary stent 128 has a distal end 130 positioned at least partially in the at least one branch vessel 14, a proximal end 132 positioned at least partially in the stent graft 100, and an intermediate portion 134 connecting the proximal and distal ends. Similar to the stent graft 100, the secondary stent 128 can have a lumen 136 that extends at least partially through the intermediate portion 134. The lumen 136 may extend through any or all of the intermediate portion 134, for example from the distal end 130 to the proximal end 132. The secondary stent 128 is configured to allow fluid communication between the at least one branch vessel 14 and the lumen 110 of the elongated body 102 of the stent graft 100. The stent graft 100 with at least one station 122 thereby facilitates the placing of a secondary stent 128. A known stent graft would often be placed such that the at least one branch vessel was occluded if the damage in the body lumen was too near the at least one branch. A known stent graft could alternatively be placed to allow communication between the lumen and the branch vessel, but the damage in the lumen would be inadequately repaired and further damage could occur or another surgery would be required to fully repair the damage. Additionally, an aneurysm or dissection could extend into the at least one branch vessel and a secondary sent can be needed for repair of that branch vessel.

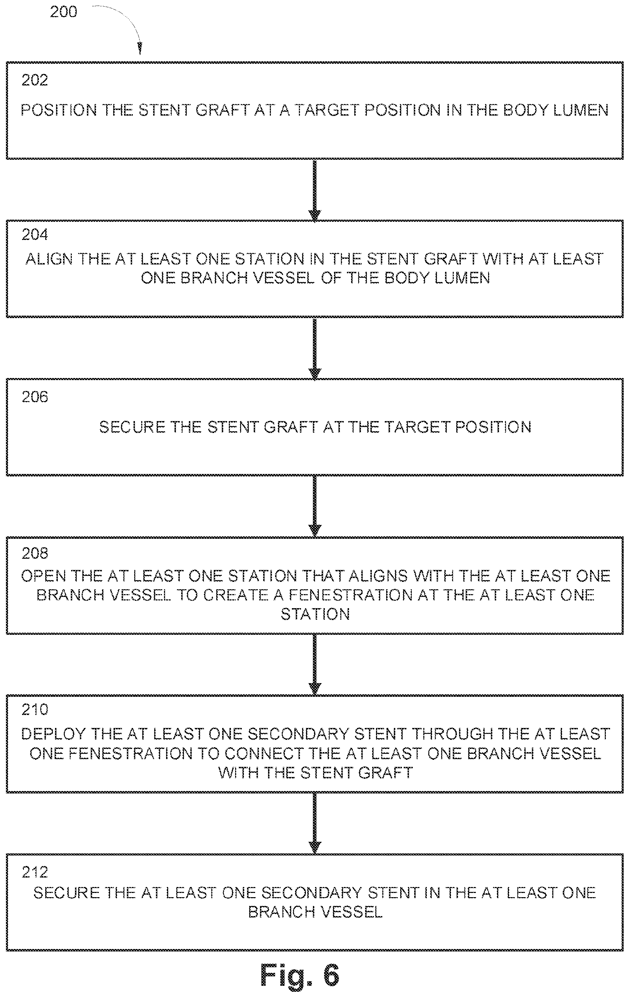

Another aspect of the present disclosure can include methods 200-700 (Figs. 6-11) for facilitating repair of a diseased or damage body lumen in a subject, such as an aneurism or dissection of the aortic arch with a stent graft, which will be presumed, for the sake of description, to be the stent graft 100 previously discussed.

The methods 200-700 are illustrated as process flow diagrams with flowchart illustrations. For purposes of simplicity, the methods are shown and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the methods.

Fig. 6 illustrates a method 200 for providing a stent graft 100, such as the stent graft 100 with at least one station 122 described above, to a body lumen of a subject. The stent graft 100 can be used to repair damage to a body lumen and can facilitate further interventions and/or stent placement in branch vessels of the body lumen. When the body lumen is part of the aorta it can be one of the aortic arch 12, the ascending aorta, the descending aorta, or the abdominal aorta. Other, non-exhaustive examples of body lumens include portions of the renal artery, the coronary artery, the carotid artery, or airways in the lungs. At step 202 the stent graft 100 can be positioned at a target position in the body lumen. The target position can be, but is not limited to, a position that covers an aneurysm, covers the tears of a dissection, blocks a hemorrhage, repairs a problem with a wall of the body lumen, or positions the stent graft 100 to facilitate other interventions, such as the placement of a secondary stent 128 in a branch vessel. At step 204 the at least one station 122 in the stent graft 100 can be aligned with the at least one branch vessel of the body lumen. The at least one station 122 can be fully or partially aligned with the at least one branch vessel, where at least part of the aperture 126 of a station 122 overlaps with at least part of an opening of a branch vessel. When the body lumen is the aortic arch 12, the at least one branch vessel 14 can be one of the brachiocephalic artery, the left common carotid artery, and the left subclavian artery.

At 206, the stent graft 100 can be secured at the target position once the at least one station 122 has been aligned. For example, the stent graft 100 can be secured via the expansion of the stent frame to appose to the vessel wall and/or with sutures, staples, suction means, a biocompatible adhesive, and/or any other securing means. The stent graft 100 can be secured such that the position of the stent graft does not substantially shift during normal bodily functions. At 208, the at least one station 122 aligned with the at least one branch vessel can be opened to create a fenestration at the at least one station. At 210, at least one secondary stent 128, such as the one described previously, can be deployed through the at least one fenestration to connect the at least one branch vessel with the stent graft 100. The secondary stent 128 can allow fluid communication between the at least one branch vessel and the lumen 110 of the stent graft 100. At 212 the at least one secondary stent 128 can be secured in the at least one branch vessel and/or the at least one station 122. The secondary stent 128 can be secured to the at least one branch vessel and the at least one station 122, for example, via the expansion of a frame of the secondary stent to appose to the vessel wall and ring structure 124, respectively, and/or with sutures, staples, an adhesive, and/or other securing means.

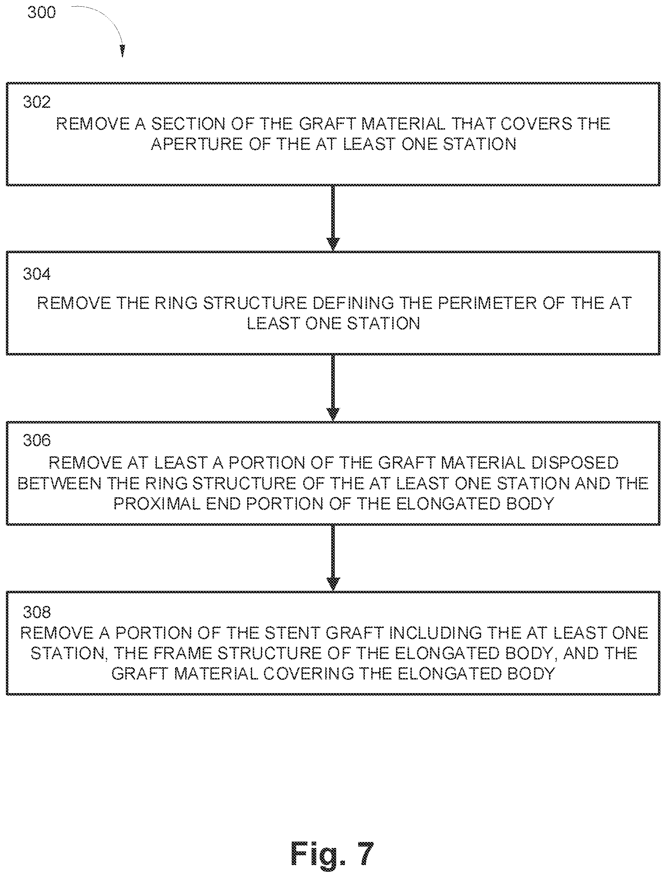

Fig. 7 illustrates a method 300 for opening the at least one station 122 of the stent graft 100 aligned with the at least one branch vessel to create a fenestration at the at least one station. Each opening step may be done alone or in combination with any of the other steps. At step 302 a section of the graft material 118 that covers the aperture 126 of the at least one station 122 can be removed to create a fenestration. Removing a section of the graft material 118 can comprise creating a puncture in the graft material, cutting through a section of the graft material, cutting out a section of the graft material, or any other suitable removal scheme. At step 304 removing the ring structure 124 defining the perimeter of the at least one station 122 can create a fenestration. Removing the ring structure 124 can include cutting out part, or all, of the ring structure. Removing the ring structure 124 can include removing the ring structure itself and removing the graft material 118 covering the aperture 126 formed by the ring structure. At step 306 removing at least a portion of the graft material 118 disposed between the ring structure 124 of the at least one station 122 and the proximal end portion 104 of the elongated body 102 can create a fenestration. In addition to removing the graft material 118 disposed between the ring structure 124 and the proximal end portion 104 of the elongated body 102, the graft material covering the aperture 126 of the at least one station 122 can be opened and/or removed and the ring structure, or any portion thereof, can also be removed. At step 308 removing a portion of the stent graft 100 including the at least one station 122, the frame structure 112 of the elongated body 102, and the graft material 118 covering the elongated body can create a fenestration. At either of steps 306 and 308 a new edge of the stent graft 100 may be created. The new edge can be used for further securing the stent graft or for other interventions such as an anastomosis.

The at least one station 122 can include at least a first station and a second station where at least one of the first station and the second station can be selectively covered by the graft material 118. A selected one of the first and second stations 122 can have an aperture 126 covered by the graft material 118 and an other one of the first and second station can have an open aperture that is not covered by the graft material. In another instance both of the first and second stations 122 can be covered by the graft material 118. Either at least one covered or non-covered station 122 can be aligned with the at least one branch vessel, as desired for a particular use environment, and the stations 122 can be selectively recovered or uncovered during performance of the method, as desired.

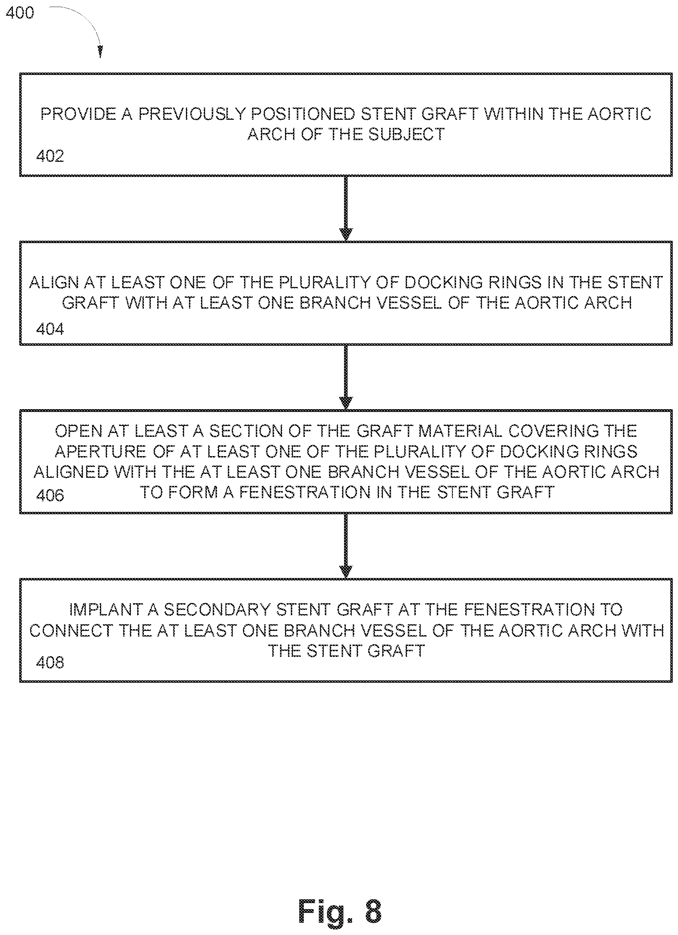

Fig. 8 illustrates a method 400 of providing a stent graft to an aortic arch 12 of a subject. At step 402 a previously positioned stent graft can be provided within the aortic arch of the subject. The stent graft can be the stent graft 100 described above with at least one station 122. For some use environments, the stent graft can be similar to the stent graft 100 described above with a plurality of docking rings in place of the at least one station 122. At step 404 at least one of the plurality of docking rings in the stent graft can be aligned with at least one branch vessel 14 of the aortic arch 12. At step 406, at least a section of the graft material 118 covering the aperture of at least one of the plurality of docking rings aligned with the at least one branch vessel 14 of the aortic arch 12 can be opened to form a fenestration in the stent graft. At step 408, a secondary stent can be implanted at the fenestration to connect the at least one branch vessel of the aortic arch with the stent graft (e.g., to allow fluid communication between the branch vessel and the lumen of the stent graft). In another method, not illustrated, instead of steps 406 and 408, the graft material covering over the apertures of the plurality of docking rings can be maintained to at least partially occlude fluid communication between the at least one branch vessel and the lumen of the stent graft. Maintaining the graft material covering can help to regulate the amount of fluid that flows from the at least one branch vessel into the stent graft, or vice versa.

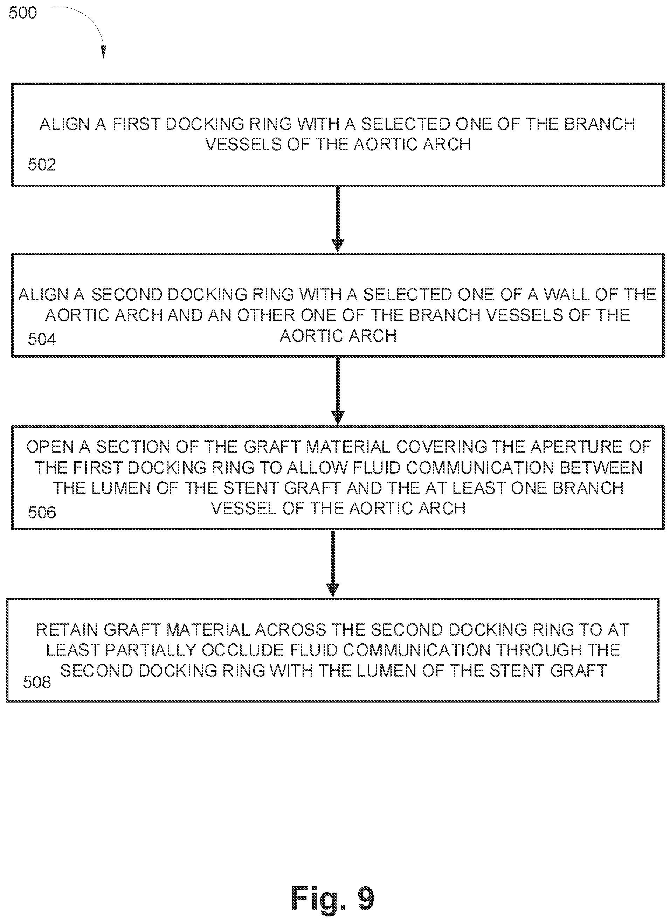

Fig. 9 illustrates a method 500 of providing a stent graft to an aortic arch of a subject, where the plurality of docking rings of the stent graft includes at least two docking rings. At step 502, a first docking ring of the plurality of docking rings of the previously provided stent graft can be aligned with a selected one of the branch vessels of the aortic arch. At step 504, a second docking ring of the plurality of docking rings can be aligned with a selected one of a wall of the aortic arch and an other one of the branch vessels of the aortic arch. At step 506 a section of the graft material covering the aperture of the first docking ring can be opened to allow fluid communication between the lumen of the stent graft and the at least one branch vessel of the aortic arch. At step 508 graft material across the second docking ring of the plurality of docking rings can be retained to at least partially occlude fluid communication through the second docking ring with the lumen of the stent graft. The graft material can be fully or partially retained.

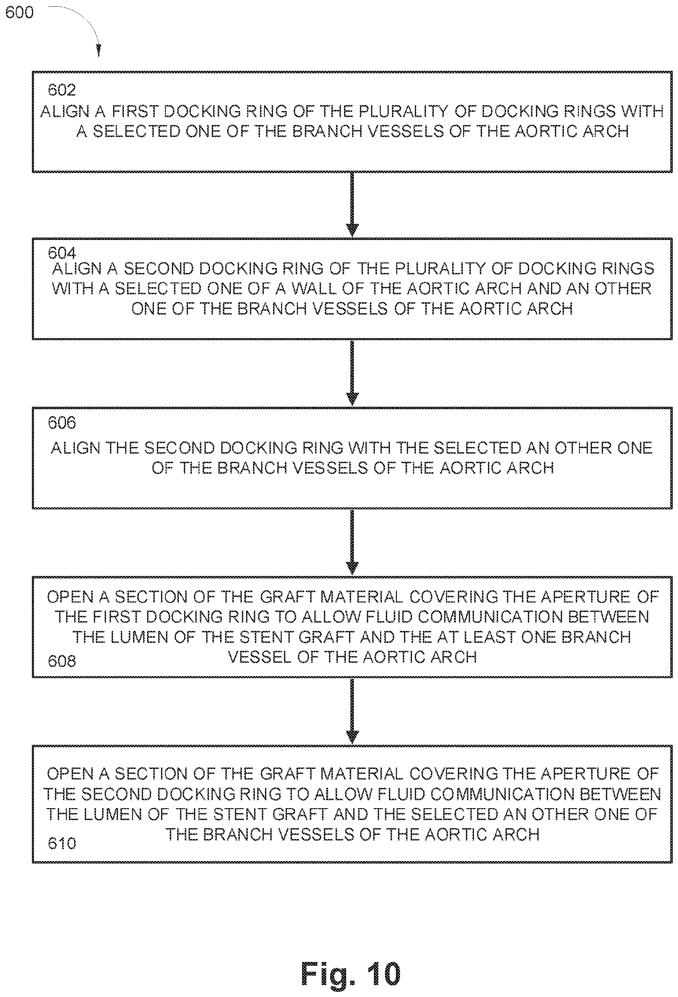

Fig. 10 illustrates a method 600 of providing a stent graft to an aortic arch of a subject, where the plurality of docking rings of the stent graft includes at least two docking rings. At step 602, a first docking ring of the plurality of docking rings of the previously provided stent graft can be aligned with a selected one of the branch vessels of the aortic arch. At step 604, a second docking ring of the plurality of docking rings can be aligned with a selected one of a wall of the aortic arch and an other one of the branch vessels of the aortic arch. At step 606, the second docking ring can be aligned with the selected one of the branch vessels of the aortic arch. At step 608, a section of the graft material covering the aperture of the first docking ring of the plurality of docking rings can be opened to allow fluid communication between the lumen of the stent graft and the at least one branch vessel of the aortic arch. At step 610, a section of the graft material covering the aperture of the second docking ring of the plurality of docking rings can be opened to allow fluid communication between the lumen of the stent graft and the selected one of the branch vessels of the aortic arch. The opened section of the graft material covering the apertures of the first and second docking rings can be all, or any part, of the graft material covering the apertures. For example, one station can be aligned with the left common carotid artery and another can be aligned with the left subclavian artery. The coverings of both stations can be opened to allow fluid communication between both the left common carotid artery and the left subclavian artery and the stent graft within the aortic arch. Secondary stents can be positioned through one of the openings, none of the openings, or both of the openings. Fluid communication may be possible through the fenestration of the docking ring without the placement of a secondary stent.

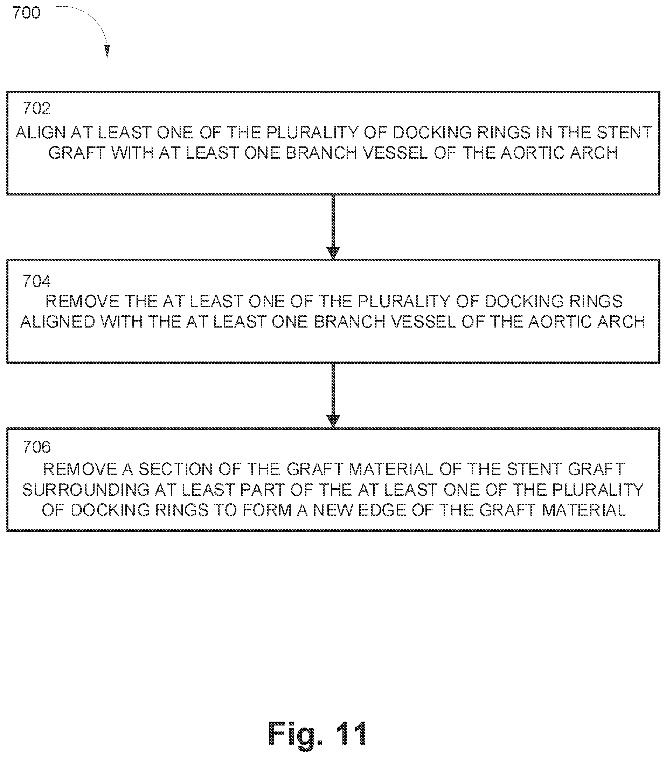

Fig. 11 illustrates a method 700 for forming an anastomosis of the previously positioned and aligned stent graft. At step 702 at least one of the plurality of docking rings aligned with the at least one branch vessel of the aortic arch can be removed. Removing the at least one of the plurality of docking rings can include removing the ring structure defining the perimeter of the docking ring and removing the graft material covering the aperture of the docking ring. At step 704, a section of the graft material of the stent graft surrounding at least part of the at least one of the plurality of docking rings can be removed to form a new edge of the graft material. A part of the frame structure of the stent graft may also be removed. At step 706, an anastomosis can be formed between the stent graft and the at least one branch vessel of the aortic arch with the new edge of the graft material.

Regardless of how the stent graft and secondary stent(s), if any, are provided to the target vessel, the stent graft may be left in place at least semi-permanently, optionally including some degree of ingrowth into the tissue of the surrounding body lumen walls. It is contemplated that a surgeon could re-access a previously installed stent graft to replace an existing secondary stent, add a new secondary stent (such as by opening another station of the stent graft), re-cover a previously fenestrated station, remove the stent graft (while optionally allowing the secondary stent to remain in place), replace the stent graft entirely, or for any other reason.

## Claims

1. A stent graft (100), comprising:
an elongated body (102) having proximal and distal end portions (104, 106) longitudinally separated by an intermediate portion (108), a lumen (110) extending at least partially through the intermediate portion (108), and a frame structure (112) having inner and outer surfaces (114, 116), at least one of the inner and outer surfaces (114, 116) being at least partially covered by a graft material (118), wherein at least one of the proximal and distal end portions (104, 106) includes an aperture (120) in fluid communication with the lumen (110) and defined by a portion of the frame structure (112); and
at least one station (122) positioned on the graft material (118) of the elongated body (102), the station (122) having a perimeter defined by a ring structure (124) connected to the graft material (118) covering the frame structure (112) and a selectively open aperture (126) bounded by the perimeter, wherein the aperture (126) of the at least one station (122) is selectively covered by the graft material (118); and
**characterised by**,
the ring structure is a ring of sutures,
a section that can be removed by fracturing and/or cutting through a part of a ring structure for forming a fenestration in the stent graft (100), the section including the at least one station (122), the ring structure (124), a portion of the frame structure (112) of the elongated body (102), and the graft material (118) at least partially covering the elongated body (102), wherein the section extends into the aperture (120) at the proximal or distal end portion (104, 106).

2. The stent graft (100) of claim 1, wherein the at least one station (122) is positioned proximate the proximal end portion (104) of the elongated body (102) to substantially align with at least one branch vessel (14) of the aorta when the stent graft (100) is located therein.

3. The stent graft (100) of claim 2, wherein the at least one branch vessel (14) is a selected one of the brachiocephalic artery (24), the left common carotid artery (26), and the left subclavian artery (28).

4. The stent graft (100) of claim 1 or claim 2, wherein at least a section of the graft material (118) covering the aperture (126) of the at least one station (122) is selectively removable to form a fenestration.

5. The stent graft (100) of claim 4, wherein the fenestration of the at least one station (122) provides a site in the stent graft (100) at which a secondary stent (128) can be introduced to the at least one branch vessel (14).

6. The stent graft (100) of claim 5, including the secondary stent (128) at least partially passing through the fenestration of the at least one station (122), the secondary stent (128) having a distal end (130) positioned at least partially in the at least one branch vessel (14) and a proximal end (132) positioned at least partially in the stent graft (100), wherein the secondary stent (128) is configured to allow fluid communication between the at least one branch vessel (14) and the lumen (110) of the elongated body (102).

7. The stent graft (100) of any of claims 1 to 4, wherein the perimeter is at least one of a rectilinear shape, a curvilinear shape, a circle, an oval, a rectangle, and a polygon.

8. The stent graft (100) of any of claims 1 to 4 or claim 7, wherein the at least one station (122) further includes at least one secondary ring structure (125) located within the perimeter defined by the ring structure (124), wherein the at least one secondary ring structure (125) is located within the ring structure (124) and defines a smaller area for creating the fenestration in the station (122).

9. The stent graft (100) of any of claims 1 to 4 or claim 7 or 8, wherein the at least one station (122) further includes at least a first station and a second station, wherein the second station has a different perimeter than the first station.

## Patentansprüche

1. Stentimplantat (100), umfassend:
einen länglichen Körper (102) mit proximalem und distalem Endabschnitte (104, 106), die in Längsrichtung durch einen Zwischenabschnitt (108) getrennt sind, einem Lumen (110), das sich mindestens teilweise durch den Zwischenabschnitt (108) erstreckt, und einer Rahmenstruktur (112) mit Innen- und Außenfläche (114, 116), wobei mindestens eine der Innen- und Außenfläche (114, 116) mindestens teilweise von einem Implantatmaterial (118) bedeckt ist, wobei mindestens einer des proximalen und des distalen Endabschnitts (104, 106) eine Öffnung (120) beinhaltet, die in Fluidverbindung mit dem Lumen (110) steht und durch einen Abschnitt der Rahmenstruktur (112) definiert ist; und
mindestens eine Station (122), die auf dem Implantatmaterial (118) des länglichen Körpers (102) positioniert ist, wobei die Station (122) einen Umfang, der durch eine Ringstruktur (124) definiert ist, die mit dem Implantatmaterial (118) verbunden ist, das die Rahmenstruktur (112) bedeckt, und eine selektiv offene Öffnung (126), die durch den Umfang begrenzt ist, aufweist, wobei die Öffnung (126) der mindestens einen Station (122) selektiv durch das Implantatmaterial (118) bedeckt ist; und
**gekennzeichnet durch**
die Ringstruktur ist ein Ring aus Nähten,
einen Abschnitt, der durch Frakturieren und/oder Schneiden durch einen Teil einer Ringstruktur zum Bilden eines Fensters in dem Stentimplantat (100) entfernt werden kann, wobei der Abschnitt die mindestens eine Station (122), die Ringstruktur (124), einen Abschnitt der Rahmenstruktur (112) des länglichen Körpers (102) und das Implantatmaterial (118) beinhaltet, das den länglichen Körper (102) mindestens teilweise bedeckt, wobei sich der Abschnitt in die Öffnung (120) an dem proximalen oder dem distalen Endabschnitt (104, 106) erstreckt.

2. Stentimplantat (100) nach Anspruch 1, wobei die mindestens eine Station (122) in der Nähe des proximalen Endabschnitts (104) des länglichen Körpers (102) positioniert ist, um sich im Wesentlichen mit mindestens einem Zweiggefäß (14) der Aorta auszurichten, wenn sich das Stentimplantat (100) darin befindet.

3. Stentimplantat (100) nach Anspruch 2, wobei das mindestens eine Zweiggefäß (14) ein ausgewähltes aus der Arteria brachiocephalica (24), der linken Arteria carotis communis (26) und der linken Arteria subclavia (28) ist.

4. Stentimplantat (100) nach Anspruch 1 oder Anspruch 2, wobei mindestens ein Abschnitt des Implantatmaterials (118), das die Öffnung (126) der mindestens einen Station (122) bedeckt, selektiv entfernbar ist, um ein Fenster zu bilden.

5. Stentimplantat (100) nach Anspruch 4, wobei das Fenster der mindestens einen Station (122) eine Stelle in dem Stentimplantat (100) bereitstellt, an der ein sekundärer Stent (128) in das mindestens eine Zweiggefäß (14) eingeführt werden kann.

6. Stentimplantat (100) nach Anspruch 5, beinhaltend, dass der sekundäre Stent (128) mindestens teilweise durch das Fenster der mindestens einen Station (122) verläuft, wobei der sekundäre Stent (128) ein distales Ende (130), das mindestens teilweise in dem mindestens einen Zweiggefäß (14) positioniert ist, und ein proximales Ende (132), das mindestens teilweise in dem Stentimplantat (100) positioniert ist, aufweist, wobei der sekundäre Stent (128) dazu konfiguriert ist, eine Fluidkommunikation zwischen dem mindestens einen Zweiggefäß (14) und dem Lumen (110) des länglichen Körpers (102) zu ermöglichen.

7. Stentimplantat (100) nach einem der Ansprüche 1 bis 4, wobei der Umfang mindestens einer von einer geradlinigen Form, einer gekrümmten Form, einem Kreis, einem Oval, einem Rechteck und einem Polygon ist.

8. Stentimplantat (100) nach einem der Ansprüche 1 bis 4 oder Anspruch 7, wobei die mindestens eine Station (122) ferner mindestens eine sekundäre Ringstruktur (125) beinhaltet, die sich innerhalb des durch die Ringstruktur (124) definierten Umfangs befindet, wobei sich die mindestens eine sekundäre Ringstruktur (125) innerhalb der Ringstruktur (124) befindet und einen kleineren Bereich zum Erzeugen des Fensters in der Station (122) definiert.

9. Stentimplantat (100) nach einem der Ansprüche 1 bis 4 oder Anspruch 7 oder 8, wobei die mindestens eine Station (122) ferner mindestens eine erste Station und eine zweite Station beinhaltet, wobei die zweite Station einen anderen Umfang als die erste Station aufweist.

## Revendications

1. Endoprothèse couverte (100) comprenant :
un corps allongé (102) possédant des parties d'extrémité proximale et distale (104, 106) séparées longitudinalement par une partie intermédiaire (108), une lumière (110) s'étendant au moins partiellement à travers la partie intermédiaire (108), et une structure de cadre (112) possédant des surfaces interne et externe (114, 116), au moins l'une des surfaces interne et externe (114, 116) étant au moins partiellement recouverte d'un matériau de greffe (118), au moins l'une desdites parties d'extrémité proximale et distale (104, 106) comprenant une ouverture (120) en communication fluidique avec la lumière (110) et définie par une partie de la structure de cadre (112) ; et
au moins une station (122) positionnée sur le matériau de greffe (118) du corps allongé (102), la station (122) présentant un périmètre défini par une structure annulaire (124) reliée au matériau de greffe (118) recouvrant la structure de cadre (112) et une ouverture ouverte de manière sélective (126) délimitée par le périmètre, ladite ouverture (126) de ladite au moins une station (122) étant recouverte de manière sélective par le matériau de greffe (118) ; et
**caractérisé en ce que**,
la structure annulaire est un anneau de sutures,
une section qui peut être retirée par fracturation et/ou coupe à travers une partie d'une structure annulaire pour former une fenestration dans l'endoprothèse recouverte (100), la section comprenant ladite au moins une station (122), la structure annulaire (124), une partie de la structure de cadre (112) du corps allongé (102), et le matériau de greffe (118) recouvrant au moins partiellement le corps allongé (102), ladite section s'étendant dans l'ouverture (120) au niveau de la partie d'extrémité proximale ou distale (104, 106).

2. Endoprothèse couverte (100) selon la revendication 1, ladite au moins une station (122) étant positionnée à proximité de la partie d'extrémité proximale (104) du corps allongé (102) de manière à s'aligner sensiblement avec au moins un vaisseau ramifié (14) de l'aorte lorsque l'endoprothèse couverte (100) s'y trouve.

3. Endoprothèse couverte (100) selon la revendication 2, ledit au moins un vaisseau ramifié (14) étant un vaisseau sélectionné parmi l'artère brachiocéphalique (24), l'artère carotide commune gauche (26) et l'artère sous-clavière gauche (28).

4. Endoprothèse couverte (100) selon la revendication 1 ou la revendication 2, au moins une section du matériau de greffe (118) recouvrant l'ouverture (126) de ladite au moins une station (122) pouvant être retirée de manière sélective de manière à former une fenestration.

5. Endoprothèse couverte (100) selon la revendication 4, ladite fenestration de ladite au moins une station (122) fournissant un site dans l'endoprothèse couverte (100) au niveau duquel une endoprothèse secondaire (128) peut être introduite dans ledit au moins un vaisseau ramifié (14).

6. Endoprothèse couverte (100) selon la revendication 5, comprenant l'endoprothèse secondaire (128) passant au moins partiellement à travers la fenestration de ladite au moins une station (122), l'endoprothèse secondaire (128) possédant une extrémité distale (130) positionnée au moins partiellement dans ledit au moins un vaisseau ramifié (14) et une extrémité proximale (132) positionnée au moins partiellement dans l'endoprothèse couverte (100), ladite endoprothèse secondaire (128) étant conçue pour permettre la communication fluidique entre ledit au moins un vaisseau ramifié (14) et la lumière (110) du corps allongé (102).

7. Endoprothèse couverte (100) selon l'une quelconque des revendications 1 à 4, ledit périmètre étant au moins un périmètre parmi une forme rectiligne, une forme curviligne, un cercle, un ovale, un rectangle et un polygone.

8. Endoprothèse couverte (100) selon l'une quelconque des revendications 1 à 4 ou la revendication 7, ladite au moins une station (122) comprenant en outre au moins une structure annulaire secondaire (125) se trouvant à l'intérieur du périmètre défini par la structure annulaire (124), ladite au moins une structure annulaire secondaire (125) se trouvant à l'intérieur de la structure annulaire (124) et définissant une zone plus petite de manière à créer la fenestration dans la station (122).

9. Endoprothèse couverte (100) selon l'une quelconque des revendications 1 à 4 ou la revendication 7 ou 8, ladite au moins une station (122) comprenant en outre au moins une première station et une seconde station, ladite seconde station présentant un périmètre différent de celui de la première station.
